Europäisches Patentamt

European Patent Office

Office européen des brevets

(11) Publication number: **0 532 833 A1**

## EUROPEAN PATENT APPLICATION

(21) Application number: **92108271.5**

(22) Date of filing: **15.05.92**

(51) Int. Cl.⁵: **A61K 39/27**

(30) Priority: **28.05.91 US 705833**

(43) Date of publication of application:
**24.03.93 Bulletin 93/12**

(84) Designated Contracting States:
**BE CH DE DK ES FR GB IT LI NL SE**

(71) Applicant: **MILES INC.**
**One Mellon Center 500 Grant Str.**
**Pittsburgh, PA 15219-2502(US)**

(72) Inventor: **Macek, Joseph**
**Rt. 5, Box 151**
**Paola, KS 66071(US)**
Inventor: **Brown, Karen K.**
**5501 N.W. Fox Hill Road Parkville**
**MO 64152(US)**
Inventor: **Greene, Nathan D.**
**15463 Overbrook Lane**
**Leawood, KS 66224(US)**
Inventor: **Smith, Richard D.**
**9310 Tomashaw Lane**
**Lenexa, KS 66219(US)**

(74) Representative: **Schumacher, Günter, Dr. et al**
**c/o Bayer AG Konzernverwaltung RP**
**Patentabteilung**
**W-5090 Leverkusen 1 Bayerwerk (DE)**

(54) **Vaccine for equine rhinopneumonitis.**

(57) A combination vaccine which is effective against Equine rhinopneumonitis is made by combining an inactivated Equine herpesvirus type 1 with an inactivated Equine herpesvirus type 4 and an adjuvant. The herpesvirus type 1 and herpesvirus type 4 are combined in ratios of from about 4:1 to about 1:1. The adjuvant is preferably Havlogen, a Carbopol acrylic-based adjuvant.

EP 0 532 833 A1

## BACKGROUND OF THE INVENTION

The present invention relates to a combination vaccine for Equine rhinopneumonitis and to a method for protecting horses against Equine rhinopneumonitis in which this vaccine is employed.

There are four recognized types or subtypes of Equine herpesvirus. Equine herpes 1, subtype 1 is also known as Equine abortion virus. Subtype 2 of EHV-1 is the Respiratory Rhinopneumonitis virus. EHV-2 is sometimes called Equine cytomegalovirus. EHV-3 is known as Equine coital exanthema virus. Recent DNA homology studies have shown significant differences between EHV-1 Subtype 1 and Subtype 2. Some scientists have therefore proposed that EHV-1, Subtype 2 be renamed EHV-4 to reflect this difference. In this application, EHV-1, Subtype 2 is referred to as EHV-4.

Rhinopneumonitis is an acute viral infection characterized by fever, leukopenia and catharral inflammation in the respiratory tract. The viral disease predisposes to secondary bacterial infection that affects various parts of the respiratory tract. Abortion is often a sequel when pregnant mares are infected.

A vaccine that is effective against rhinopneumonitis has long been sought. U.S. Patent 4,110,433, for example, discloses a vaccine for rhinopneumonitis that is an attenuated form of the virus prepared from a virulent strain of Equine Rhinopneumonitis Virus that had been passaged through the Vero Cell line (monkey kidney) at 26°C for a sufficient number of passes under conditions such that the virus became attenuated to an avirulent condition without losing its immunogenic character. The vaccine disclosed in this patent is not, however, inactivated. Since it is a live vaccine, the virus could revert back to virulence. In fact, soon after this vaccine was released for sale it was withdrawn from the market because the "avirulent" vaccine produced disease in horses that, in some cases, resulted in death.

U.S. Patent 4,083,958 discloses an inactivated Equine rhinopneumonitis virus vaccine that is prepared by propagating an Equine rhinopneumonitis virus in a susceptible, cloned, diploid Equine cell line, harvesting the resulting virus in a serum-free medium, chemically inactivating the virus and neutralizing the inactivation agent. The resultant virus is concentrated and an immunologic adjuvant is added. The resultant vaccine is then administered to young female horses at regular intervals until they become pregnant and during their pregnancy. This disclosed vaccine is not, however, a combination of two Subtypes of Equine herpesvirus type 1. The vaccine of the present invention contains EHV-1 and EHV-4.

U.S. Patent 4,225,582 also discloses a vaccine for Equine rhinopneumonitis. Unlike the above-discussed vaccines, however, this vaccine is a live bovine herpesvirus [A.T.C.C. VR-2003]. This strain was tested in our laboratories and found to produce abortion in pregnant mares. Restriction endonuclease analysis has shown the strain used to produce this disclosed vaccine to be a typical wild EHV-1.

U.S. Patent 4,466,957 discloses adjuvants useful in vaccines based upon killed organisms such as Equine rhinopneumonitis virus. This patent does not, however, teach the use of a polyacrylic compound such as Carbopol as an adjuvant in the vaccine.

## SUMMARY OF THE INVENTION

It is an object of the present invention to provide a new combination vaccine that is effective in the prevention of Equine rhinopneumonitis and Equine abortion.

It is also an object of the present invention to provide a new combination vaccine that is formed from inactivated viruses and is effective in the prevention of Equine rhinopneumonitis and Equine abortion.

It is another object of the present invention to provide a combination vaccine that is more effective in preventing Equine rhinopneumonitis virus infection than known vaccines for Equine rhinopneumonitis virus.

It is a further object of the present invention to provide a method for treating horses to protect them against Equine rhinopneumonitis virus and abortion attributable to that virus.

These and other objects which will be apparent to those skilled in the art are accomplished by combining inactivated Equine herpesvirus type 1 and inactivated Equine herpesvirus type 4 and an adjuvant in quantities such that the weight ratio of inactivated Equine herpesvirus type 1 to inactivated Equine herpesvirus type 4 is from about 4:1 to about 1:1, preferably from about 3:1 to about 2:1. It i necessary to differentiate between Equine herpesvirus type 1 and Equine herpesvirus type 4 to produce the vaccine of the present invention. ELISA technology in an antigen capture mode makes it possible to detect and quanitate an antigen on the Equine herpesvirus type 4 which confers protective immunity upon vaccination of horses. This vaccine may be administered to a horse by any of the known techniques, preferably by intramuscular injection.

## DETAILED DESCRIPTION OF THE INVENTION

### A. Vaccine Viruses

DA-8 Virus - The original seed of the isolate of EHV-1 which we have designated DA-8 was obtained from Dr. S. Dutta at the University of Maryland. Dr. Dutta has isolated DA-8 from an aborted foal. This isolate was less virulent than the isolate DA-35 (discussed infra) as evidenced by vaccination, challenge and Protection of hamsters as well as Protection of horses from challenge. The original seed was subsequently passaged 4 times in Equine Dermis Cells (ED) in accordance with techniques known to those in the art to make Master Seed Virus. Working seed was made by passaging Master Seed Virus in ED cells (i.e., the fifth passage in ED cells of the original seed).

The ED cells used to passage the EHV-1 seed were cells obtained from the ATCC designated ATCC CCC57 which had been passaged approximately 40 times in accordance with known technology. ED cells were used because they are host related and are therefore less likely to produce a reaction in the horses vaccinated. It would be possible to passage the EHV-1 seed in other cell lines such as Madin Darby Bovine Kidney (MDBK) and Vero (Monkey kidney). However, because these cell lines are not derived from horses, some reaction may occur in horses vaccinated with a vaccine derived from EHV-1 passaged in those cell lines.

The virus used to produce the vaccine was a 20 liter lot made from the Working Seed (obtained after the 5th passage in ED cells from the original seed received from Dr. Dutta). The titer of the lot was $10^{6.0}$ 50% Tissue Culture Infective Doses per ml ($TCID_{50}$/ml). A 3 liter portion of this lot was saved for the nonconcentrated vaccine. The remaining 17 liters of this virus lot were allowed to settle for 7 days at 4°C. The top portion was then carefully siphoned off so as to not disturb the settled out cell debris. The cell free virus suspension was concentrated using an Amicon $CH_2$ ultrafiltration unit with a 30,000 Dalton molecular weight cutoff spiral wrap cartridge. A concentrate that was ten times more concentrated than the initial suspension (hereinafter referred to as "10X concentrate") was produced in this manner. The settled ED cell debris was resuspended in the 10X concentrate resulting in a titer of $10^{6.8}$ $TCID_{50}$/ml.

DA-35 Virus - The original seed strain of EHV-1 that we have designated DA-35 was obtained from Dr. S. Dutta of the University of Maryland. This seed which was isolated from an aborted foal was more virulent than isolate DA-8 as evidenced by the results of vaccination, challenge and Protection tests done with hamsters and horses. Following the same procedure described above with respect to the DA-8 isolate, Master Seed Virus and Working Seed were prepared by passage on Equine dermal (ED) cells.

The virus used to produce the vaccine was a 41 liter lot made from the Working Seed (obtained after the 5th passage in ED cells from the original seed received from Dr. Dutta). The titer of the lot was $10^{7.0}$ $TCID_{50}$/ml. A 3 liter portion of this lot was saved for the nonconcentrated vaccine. A 29 liter portion of this lot was allowed to settle for 7 days at 4°C, the top portion was siphoned off and concentrated to ten times its original concentration using the same type of ultrafiltration procedure as described above. The ED cell debris was resuspended in the 10X concentrate resulting in a titer of $10^{8.2}$ $TCID_{50}$/ml.

EHV-4 Virus - It is necessary to differentiate between Equine herpesvirus type 1 and Equine herpesvirus type 4 to produce the vaccine of the present invention. ELISA technology in an antigen capture mode makes it possible to detect and quantitate an antigen on the Equine herpesvirus type 4 virus which confers protective immunity upon vaccination of horses. The original seed of EHV-4 used to produce the vaccine of the present invention was obtained from Dr. Pearson at the National Veterinary Service Laboratory (NVSL -Ames, Iowa). The vial was labeled Equine Herpes 1 isolate Subtype 2, 761036-1, 8/27/82. The seed was isolated by Dr. Pearson from a horse diagnosed with Equine rhinopneumonitis. The original seed was passaged 5 times in ED cells in accordance with techniques known in the art to make Master Seed Virus.

The virus used to make the vaccine was a 20 liter lot made from the working seed that was obtained after the 6th passage in ED cells from the original seed received from Dr. Pearson. The titer of the lot was $10^{5.9}$ $TCID_{50}$/ml. A 3 liter portion of the lot was saved for the nonconcentrated vaccine and the remaining 17 liters of the lot were concentrated 10X (titer $10^{7.0}$ $TCID_{50}$) by the method previously described for EHV-1 DA-35.

Table 1 summarizes the vaccine viruses showing virus strain, cell substrate used for virus propagation and virus $TCID_{50}$ titers of nonconcentrated virus and the 10X concentrate.

B. Vaccines

Each of the 6 lots of virus prepared in accordance with the procedures described above (i.e., EHV-1 (DA-8 and its concentrate, EHV-1 (DA-35) and its concentrate and EHV-4 and its concentrate) was used to prepare the vaccine formulations in accordance with the procedure described below.

Each of the six lots of virus samples was inactivated with beta propiolactone (BPL). The BPL was diluted 1:10 in ice cold distilled water. An amount of this BPL solution sufficient to produce a final BPL concentration of 0.15% was added to each virus sample at 4°C. The temperature was then slowly raised to 37°C (over approximately 1 hour). The samples were held at 37°C to hydrolyze any residual BPL. During this hydrolysis, 5N NaOH was added as needed to keep the pH at neutrality as determined by the indicator, phenol red, in the medium. The hydrolysis was complete in approximately 5 hours. The virus samples were stored at 4°C until inactivation assurance testing was completed.

To test for complete inactivation of the viruses, a 10 ml aliquot of BPL treated virus was used to inoculate a 75 $cm^2$ tissue culture flask of ED cells. The cells were monitored for 1 week for signs of cytopathic effect (CPE). One week after inoculation, if no CPE was observed, the cells were subcultured and observed for an additional week. If at the end of the second week no CPE was observed, the virus was deemed inactivated.

Each of the virus samples was separately adjuvanted with a Carbopol polyacrylic-based adjuvant sold under the name Havlogen to stimulate T cell and B cell response. A suitable adjuvant must not be reactive (horses are very sensitive). The Havlogen adjuvant used in the vaccines of the present invention described infra was added in an amount such that the final concentration was 10% (0.25% Carbopol polyacrylic).

These Havlogen adjuvant containing virus samples were used to prepare the vaccines described in Table 2. Each vaccine was formulated as a 2.0 ml dose to be given intramuscularly.

C. Experimental Animals

Sixty horses were used to study the efficacy of the vaccine of the present invention. Each horse was assigned to one of 4 groups so that the groups were balanced, as nearly as possible, in terms of EHV antibody titers and source of the horses. Twenty horses were assigned to each of Groups 1 and 2; Groups 3 and 4 were each assigned 10 animals. One of the horses which had been assigned to Group 4 was removed from the study prior to challenge due to an unrelated infection. There were therefore only 9 controls. On the ninth day after challenge, one of the horses in the EHV-1 (DA-35) concentrate/EHV-4 concentrate vaccination group, ruptured herself by jumping over the chute and landing with her abdomen on the rear gate. She died as a result of this injury. There were therefore no results for this horse on days 10 or 11 postchallenge.

D. Vaccination

The 60 horses used in this study were divided into 4 groups as described above. Each animal was vaccinated according to its group assignment as follows:

Group 1     Vaccine B (as described in Table 2), 2 intramuscular (IM) doses (2 ml each), 3 weeks apart, administered in the neck.

Group 2     Vaccine A (as described in Table 2), 2 IM doses (2 ml each) 3 weeks apart, administered in the neck.

Group 3     Vaccine C (as described in Table 2), 2 IM doses (2 ml each) 3 weeks apart, administered in the neck.

Group 4     - Nonvaccinated controls.

E. Challenge of Horses with EHV

Challenge Virus - A highly virulent original seed of EHV-1 (MSU) was received from Dr. C. W. Purdy at Mississippi State University on 2/26/79. The virus used in the challenge was the second passage on Equine dermal (ED) cells. The challenge pool was harvested and frozen. Its titer was $10^{6.0}$ $TCID_{50}$/ml.

Each horse was challenged with approximately $10^{5.3}$ $TCID_{50}$ of EHV-1 (MSU) (1:5 dilution of the challenge virus at $10^{6.0}$ $TCID_{50}$). The virus was administered intranasally as an aerosol produced by a nebulizer (Cadema Medical Products, Middleton, New York). One ml of the 1:5 diluted challenge virus was placed in the nebulizer. A section of 1/2 inch I.D. (inner diameter) silicone tubing approximately 2 ft. long was attached to the outlet port of the nebulizer. A pressure hose was fitted from an air compressor to the inlet port of the nebulizer.

Rompun (xylazine), a sedative and analgesic, was administered intravenously to each horse prior to challenge at a dosage of 0.5 ml per 100 lbs. of body weight (0.5 mg/lb.) This was done to minimize the risk of discomfort or injury to horses or personnel. The tube from the outlet port of the nebulizer was then inserted in the horse's nostril and 10 psi of air pressure applied to the inlet port for five minutes. During this time, one ml of virus fluids was aerosolized directly into the nostril.

F. Clinical Evaluation

Observations were recorded daily. The observation procedure consisted of the following steps:
1. Monitor rectal temperature.
2. Observe nasal passages for discharge or inflamed mucosa.
3. Listen to lung sounds.
4. Observe eyes for conjunctivitis, mattering, or other signs of disease.
5. Draw a blood sample and fill two tubes that contain ethylene diamine tetraacetic acid (EDTA) as an anticoagulant (purple stoppered tubes). One sample is used to measure the white blood cell (WBC) count and the second to attempt to isolate virus from the buffy coat.
6. On the day of challenge and on the last day of the study (2 weeks post challenge) 30 ml of blood was collected from each horse and allowed to clot. The serum was used to test for increase in antibody titer, using the serum neutralization procedure.

The daily rectal temperature in degrees F of each horse was recorded. The daily rectal temperature of each horse expressed as the number of Fahrenheit degrees above baseline was then determined. The baseline was the mean of the temperatures observed on the day of challenge and the two days before challenge. Temperatures that were less than the baseline were recorded as zero. Table 3 shows the mean of the elevations above baseline temperature of the horses in each group on each day of the postchallenge observation period. On day 2 after challenge, the mean temperature of the horses vaccinated with vaccine B (as described in Table 2) was significantly lower than that of the control horses ($p \leq 0.05$).

The severity of the discharge observed in each horse on each day of the study was recorded. Each horse was monitored for nasal discharge for a period of 11 days after challenge. In the group vaccinated with vaccine B, slight discharge was observed in 5 of 220 observations (2.3%). In the group vaccinated with vaccine A, 3 of 220 (1.3%) of the observations were "slight discharge". In the group vaccinated with vaccine C the discharge was slight in 3 of 110 observations (2.7%) and moderate in 1 of 110 (0.9%). In the control group, the discharge was slight in 7 of 99 observations (7.1%), moderate in 2 of 99 (2.0%) and heavy in 2 of 99 (2.0%).

The control group proved to have significantly more discharge than any of the other groups ($p \leq 0.05$).

G. Measurement of WBC Count

The WBC (white blood cell) count was measured using a Coulter Counter model ZBI (available from Coulter Electronics, Inc., Hialeah, Florida). A 1:495 dilution of blood was made in an ISOTON II (also available from Coulter Electronics, Inc.). Red blood cells were lysed by adding ZAP-OGLOBIN II*, a reagent supplied by Coulter Electronics used to remove erythrocytes from blood to allow counting the white blood cells resulting in a final 1:500 dilution. The diluted sample was then counted on the Coulter ZBI. Results were expressed as cells per $mm^3$. Because the counts were all less than 20,000 cells/$mm^3$, no correction was made for the passage of more than one cell through the aperture at a time.

The leukocyte count was measured for each horse on each day of the study. The counts were expressed as the number of cells per cubic mm of blood. The leukocyte counts as a percent of baseline were then determined. The baseline was the mean WBC count measured on the day of challenge and the two days before challenge for each horse. Any values greater than 100% were recorded as 100%. Table 4 shows the mean of the WBC counts of all horses in each group expressed as percentages of the individual baselines. On day 5 after challenge there was significantly less leukopenia in the horses vaccinated with vaccine B (83% of baseline = 17% WBC depression) than in the nonvaccinated controls (68% of baseline = 32% WBC depression) ($p \leq 0.05$).

H. Isolation of EHV from Buffy Coat

Buffy coats were removed from whole blood samples and inoculated into flasks containing Equine dermal cells. Flasks were incubated at 37°C for 1 week and monitored on alternate days. Flasks showing

5

no cytopathic effect were negative for virus isolation.

Using the same type of calculations as were used for nasal discharge (i.e. occurrences per total observations) the following pattern was seen. In the group vaccinated with vaccine B, virus was isolated in 7 of 220 post-challenge attempts (3.2%). The ratio of isolations per attempt for the group vaccinated with vaccine A, the group vaccinated with vaccine C and the nonvaccinated controls were 5/220, 8/110, and 4/99 respectively. These correspond to 2.3%, 7.3% and 4.0%.

I. Isolation of EHV from Nasal Swabs

Five inch sterile cotton tipped swabs were used to collect a sample of nasal secretions from each horse. Each swab was immediately submerged in 3 ml of transport medium (Minimal Essential Medium with Earles Salts (MEME) and sent to the laboratory. Samples were prepared in accordance with techniques known to those in the art and inoculated individually onto ED cell monolayers. Cells were observed on alternate days for 1 week for signs of cytopathic effect (CPE).

Virus was isolated from the nasal passages of the horses in the group vaccinated with vaccine B 2 times out of 220 attempts (0.9%). In the group vaccinated with vaccine A, the frequency of isolations was 6 out of 220 (2.7%). In the group vaccinated with vaccine C, the frequency was 3/110 (2.7%). The frequency of isolations from the nonvaccinated horses was 13/99 (13.1%).

J. Serological Evaluations

The serological response to vaccination and challenge was measured using the micro-serum neutralization method known in the art. The virus used in the serum neutralization test was EHV-1 (MSU). This strain was chosen because it was the strain used for challenge.

Serum neutralization titer was calculated using the method of Reed and Muench and described in Diagnostic Procedures for Viral and Rickettsial Infections. Fourth Edition 1969, Edwin H. Lennette, Natholie J. Schmidt, Pgs. 46-52.

A summary of the geometric mean titers of the serum neutralization tests is presented below:

| Vaccine | Prevacc. | 2 Week Post Vacc. | 3 Week Post Vacc. (Pre Boost) | 2 Week Post Boost | 2 Week Post Challenge |
|---|---|---|---|---|---|
| B | 11 | 114 | 92 | 67 | 211 |
| A | 10 | 92 | 69 | 61 | 272 |
| C | 9 | 95 | 71 | 50 | 277 |
| Nonvacc. Controls | 7 | 5 | 5 | 5 | 200 |

All statistics were calculated using the Statistical Analysis System (SAS), a commonly used computerized statistics program available from SAS Institute, SAS Circle, Cary, NC 27512. The Analysis of Variance (ANOVA) procedure was used to determine if there were statistically significant differences between the 4 groups on any day of the study. If a difference was detected by ANOVA then the data for that day was evaluated by Tukey's Studentized Range (HSD) Test to determine which groups were significantly different. Significance was tested at the 95% confidence level ($p \leq 0.05$).

DISCUSSION OF RESULTS

The data show that there is some evidence of protection with each of the vaccines, but that vaccine B provides protection superior to that provided by the other two vaccines.

The elevated temperatures which are seen with an EHV-1 (MSU) challenge appear the day following challenge and remain for only 2 or 3 days. This was the case in this challenge. However, on the second day after challenge the mean temperature of the group of horses vaccinated with vaccine B was significantly lower than that of the nonvaccinated controls. Table 3 shows that the mean temperatures of the horses vaccinated with vaccines A and C were also lower than the controls; in fact the group vaccinated with vaccine A showed the same amount of temperature elevation as did the group vaccinated with vaccine B. Due to the greater variance in the former group, the difference from the controls was not statistically significant.

Very little nasal discharge was observed in this study. Although there appears to be little difference between the groups, the only horse judged to have a heavy discharge was one of the nonvaccinated controls. The difference between the nonvaccinated controls and the three vaccinated groups is statistically significant.

The frequency of isolation of virus from the buffy coat preparations was low (3.7% of postchallenge attempts yielded virus isolation). There was little difference in the frequency of isolation from horses in different groups. The percentage of positive attempts were 3.2%, 2.3%, 7.3% and 4.0% for the groups vaccinated with vaccine B, vaccine A, vaccine C and nonvaccinated controls respectively.

There was a significant difference in mean leukocyte depression between the group of horses vaccinated with vaccine B and the nonvaccinated animals. As indicated above, on the fifth day after challenge there was significantly less leukopenia in the horses vaccinated with vaccine B than in the controls (p $\leq$ 0.05). Although this difference in leukopenia was statistically significant on only the one day, Table 4 shows that during the period from day 3 post challenge through day 7 post challenge the horses vaccinated with vaccine B had consistently less leukopenia than did the control animals. The horses vaccinated with vaccines A and C also seemed to have less severe leukopenia than did the control animals, but at no time were these differences statistically significant.

One of the most impressive differences between vaccinates and controls was in virus shedding from the nasal passages. Virus was isolated from 8 of 9 controls compared with only 2 of 20 of the animals vaccinated with vaccine B. Virus was isolated from each of the 2 vaccinates on only one day, so the average number of days virus was shed by this vaccinate group was 0.1 days. The average number of days virus was shed by controls was 1.4 days (13/9). The following table summarizes the frequency of isolation and shows the percent reduction of virus shedding in each of the vaccinated groups as compared to the nonvaccinated controls.

| Vaccine Group | Frequency (isolations per attempt) | | Reduction* |
|---|---|---|---|
| B | 2/220 | (0.9%) | 93% |
| A | 6/220 | (2.7%) | 79% |
| C | 3/110 | (2.7%) | 79% |
| Nonvaccinated | 13/99 | (13.1%) | |

$$* \text{ Percent Reduction} = \frac{13.1 - P}{13.1} \times 100$$

where P = the percentage attempts which yielded virus isolation in each vaccine group.

The results of serum neutralization testing on these horses show:

1) The nonvaccinated controls remained low-titered until after challenge, indicating that the horses were not exposed to live EHV prior to challenge.

2) There appeared to be no increase in titer as a result of the booster dose. The reason for this may be that the booster dose was given soon after the first immunization.

Table 1

| TCID$_{50}$ TITERS OF EHV VIRUS | | |
|---|---|---|
| VIRUS | CELL LINE | TCID$_{50}$ TITER/ml |
| DA-8 (nonconcentrated) (10X) | E.D. | $10^{6.0}$ $10^{6.8}$ |
| DA-35 (nonconcentrated) (10X) | E.D. | $10^{7.0}$ $10^{8.2}$ |
| EHV-4 (nonconcentrated) (10X) | E.D. | $10^{5.9}$ $10^{7.0}$ |

TABLE 2

EHV VACCINES

Volumes of Each Component Per Dose

| VACCINE DESIGNATION | EHV-1 (DA-35) | EHV-4 | EHV-1 (DA-8) Concentrate | EHV-1 (DA-35) Concentrate | EVH-4 Concentrate | TOTAL DOSE VOLUME |
|---|---|---|---|---|---|---|
| C | | | 0.67 | 0.67 | 0.67 | 2.0 |
| B | | | | 1.34 | 0.67 | 2.0 |
| A | 1.34 | 0.67 | | | | 2.0 |

EP 0 532 833 A1

TABLE 3
EHV VACCINATION/CHALLENGE STUDY
MEAN TEMPERATURE
INCREASE OVER BASELINE

| Vaccine | Day 1 | Day 2 | Day 3 | Day 4 | Day 5 | Day 6 | Day 7 | Day 8 | Day 9 | Day 10 | Day 11 |
|---|---|---|---|---|---|---|---|---|---|---|---|
| B | 2.1 | 1.8 | 0.3 | 0.3 | 0.5 | 0.9 | 0.4 | 0.3 | 0.5 | 0.1 | 0.2 |
| A | 2.3 | 1.8 | 0.5 | 0.1 | 0.5 | 1.2 | 0.4 | 0.2 | 0.3 | 0.2 | 0.2 |
| C | 2.6 | 2.8 | 0.9 | 1.0 | 0.8 | 1.6 | 1.0 | 0.4 | 0.6 | 0.1 | 0.3 |
| NonVac-cinated Controls | 2.4 | 3.5 | 1.2 | 0.5 | 0.4 | 1.0 | 0.2 | 0.1 | 0.3 | 0.2 | 0.3 |

### TABLE 4
### EHV VACCINATION / CHALLENGE STUDY
### MEAN WBC COUNTS (% OF BASELINE)
### VALUE ≥ 100% EVALUATED AS 100%

| Vaccine | Day 1 | Day 2 | Day 3 | Day 4 | Day 5 | Day 6 | Day 7 | Day 8 | Day 9 | Day 10 | Day 11 |
|---|---|---|---|---|---|---|---|---|---|---|---|
| B | 91 | 89 | 83 | 78 | 83 | 82 | 84 | 82 | 90 | 93 | 97 |
| A | 89 | 80 | 80 | 75 | 81 | 77 | 80 | 77 | 85 | 89 | 94 |
| C | 92 | 87 | 82 | 78 | 81 | 79 | 83 | 82 | 89 | 93 | 94 |
| Nonvaccinated Controls | 84 | 86 | 71 | 72 | 68 | 68 | 79 | 82 | 89 | 92 | 92 |

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. A vaccine for Equine rhinopneumonitis comprising
   a) inactivated Equine herpesvirus type 1,
   b) inactivated Equine herpesvirus type 4, and
   c) an adjuvant
   in which the weight ratio of a) to b) is from about 4:1 to about 1:1.

2. The vaccine of Claim 1 in which the ratio of a) to b) is from about 3:1 to about 2:1.

3. The vaccine of Claim 1 in which the ratio of a) to b) is about 2:1.

4. The vaccine of Claim 1 in which the Equine herpesvirus type 1, has been inactivated with beta-propiolactone.

5. The vaccine of Claim 4 in which the Equine herpesvirus type 4 has been inactivated with beta-propiolactone.

6. The vaccine of Claim 5 in which the adjuvant is a water-soluble vinyl polymer based adjuvant.

7. The vaccine of Claim 1 in which the Equine herpesvirus type 4 has been inactivated with beta-propiolactone.

8. The vaccine of Claim 1 in which the adjuvant is Havlogen a Carbopol polyacrylic-based adjuvant.

9. The vaccine of Claim 1 in which the herpesvirus type 1 is the strain EHV-1 subtype 1 characterized by inducing Protection in both hamsters and horses in vaccination challenge tests.

10. The vaccine of Claim 1 in which the herpesvirus type 4 is the strain EHV-1 subtype 2 characterized by having a relative potency of at least 1.0 when compared with a known Protective vaccine.

11. A method for protecting a horse against respiratory rhinopneumonitis virus comprising administering the vaccine of Claim 1 to the horse.

12. The method of Claim 11 in which the vaccine is administered to the horse by intramuscular injection in the neck.

13. The method of Claim 11 in which the vaccine is administered in two doses spaced at least three weeks apart.

14. The method of Claim 11 in which the vaccine is administered in 2 ml doses.

European Patent
Office

PARTIAL EUROPEAN SEARCH REPORT
which under Rule 45 of the European Patent Convention
shall be considered, for the purposes of subsequent
proceedings, as the European search report

Application Number

EP  92 10 8271

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | US-A-5 084 271  (M.J. STUDDERT) <br> * claims 2-7 * <br> --- | 1-10 | A 61 K  39/27 |
| X | BIOLOGICAL ABSTRACTS abstract no. 79031277, Philadelphia, PA, US; D.R. FITZPATRICK et al.: "Immunologic relationships between equine herpes virus type I, equine abortion virus, and type 4, equine rhinopneumonitis virus" & AMERICAN JOURNAL OF VETERINARY RESEARCH, vol. 45, no. 10, 1984, pages 1947-1952 <br> * the whole abstract * <br> ---                                    -/- | 1-10 | |

TECHNICAL FIELDS
SEARCHED (Int. Cl.5)

A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :   1-10
Claims searched incompletely :   —
Claims not searched :  11-14
Reason for the limitation of the search:

Method for treatment of the human or
animal body by therapy (Article 52 (4)
EPC).

| Place of search | Date of completion of the search | Examiner |
|---|---|---|
| BERLIN | 10-09-1992 | AVEDIKIAN P.F. |

European Patent Office

PARTIAL EUROPEAN SEARCH REPORT

Application Number

EP 92 10 8271

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| X | BIOLOGICAL ABSTRACTS abstract no. 91256894, Philadelphia, PA, US; F. BURKI et al.: "Year-round antibody profile of groups of horses of a herd kept in isolation after differently terminating use of an experimental viral combination vaccine" & DEUTSCHE TIERARZTLICHE WOCHENSCHRIFT, vol. 98, no. 3, March 1991, pages 82-89 * the whole abstract * | 1-10 | |
| X | BIOLOGICAL ABSTRACTS abstract no. 76049323, Philadelphia, PA, US; T.M. CAMPBELL et al.: "Immunogenicity of equine herpesvirus type 1 and equine rhinovirus type 1 following inactivation by beta-propiolactone and UV light" & VETERINARY MICROBIOLOGY, vol. 7, no. 6, 1982, pages 535-544 * the whole abstract * | 4,5,7 | TECHNICAL FIELDS SEARCHED (Int. Cl.5) |
| A | BIOLOGICAL ABSTRACTS abstract no. 88126718, Philadelphia, PA, US; S. GOUBAU et al.: "Immunization of cattle against modified peptides of gonadotropin releasing hormone conjugated to carriers effectiveness of Freund's and alternative adjuvants" & THERIOGENOLOGY, vol. 32, no. 4, 1989, pages 557-568 * the whole abstract * | 6,8 | |